# EUROPEAN PATENT APPLICATION

(11) **EP 3 957 303 A1**
(43) Date of publication of application: **23.02.2022**
(21) Application number: 20792077.8
(22) Date of filing: 07.04.2020
(51) Int. Cl.: A61K 9/24, A61K 9/20, A61K 47/12, A61K 47/20, A61K 31/397, A61K 31/4178, A61K 31/4422, A61K 31/505

(54) **PHARMACEUTICAL COMBINATION PREPARATION COMPRISING EZETIMIBE AND LOSARTAN**

(30) Priority: 18.04.2019 KR 20190045295
(71) Applicant: Hanmi Pharm. Co., Ltd., Hwaseong-si, Gyeonggi-do 18536 (KR)
(72) Inventor: CHO, Hyuk Jun, Suwon-si, Gyeonggi-do 16358 (KR); KIM, Min Wook, Suwon-si, Gyeonggi-do 16359 (KR); IM, Ho Taek, Yongin-si, Gyeonggi-do 16909 (KR); KIM, Yong Il, Gwacheon-si, Gyeonggi-do 13835 (KR)
(74) Representative: dompatent von Kreisler Selting Werner - Partnerschaft von Patent- und Rechtsanwälten mbB
(86) International application number: PCT/KR2020/004694
(87) International publication number: WO 2020/213868

(57) **Abstract**

The present invention relates to a pharmaceutical combination preparation comprising ezetimibe and losartan. When the pharmaceutical combination preparation is dissolved in a solution of pH 4.0 to 5.0 for 10 minutes, the dissolved amount of the losartan is 5,700 µg or less, and the dissolved amount of the ezetimibe is 270µg or more. The dissolution in a solution of pH 4.0 to 5.0 is carried out under the conditions of a temperature of 37 °C and a paddle method of 50 rpm in 500 mL of acetate buffer according to United States Pharmacopoeia for the pharmaceutical combination preparation comprising 20 mg of ezetimibe and 183 mg of losartan.

## Description

### [Technical Field]

The present invention relates to a pharmaceutical combination preparation comprising ezetimibe and losartan. More specifically, the present invention relates to a pharmaceutical combination preparation comprising ezetimibe and losartan, in which the dissolution between each drug is minimally affected.

The present application claims the benefit of priority based on Korean Patent Application No. 10-2019-0045295 filed on April 18, 2019, all the contents of which are incorporated herein by reference.

### [Background Art]

Losartan is the generic name of 2-butyl-4-chloro-1-[2'-(1H-tetrazol-5-yl)[1,1'-biphenyl]-4-yl]methyl]-1H-imidazole-5-methanol and disclosed in US Patent Nos. 5,608,075, 5,138,069, 5,153,197, and the like, and in particular losartan calcium is currently commercially available under the brand name Cozaar^{®}. Losartan is used to treat hypertension and heart failure, to treat ischemic peripheral circulatory disorder and myocardial ischemia (angina pectoris), to prevent the progression of heart failure after myocardial infarction, and to treat diabetic neuropathy, glaucoma, and the like, by blocking the binding of angiotensin II, which is a vasoconstrictor substance, to its receptor.

Ezetimibe is an azetidinone derivative, has the chemical name of 1-(4-fluorophenyl)-3(R)-[3-(4-fluorophenyl)-3(S)-hydroxypropyl]-4(S)-(4-hydroxyphenyl)-2-azetidinone, is used as a drug that inhibits cholesterol absorption in the treatment and prevention of arteriosclerosis, and is also used as a combination with a statin drug that inhibits cholesterol synthesis in the liver. Such ezetimibe is a material that is practically insoluble in water, thereby exhibiting low solubility in an aqueous medium such as gastric juice, and thus low bioavailability upon oral administration.

In order to effectively treat cardiovascular diseases, combination preparations using different mechanisms such as losartan and ezetimibe are increasingly required in the clinical field, but the dissolution rate and stability are likely to be lowered due to the interaction between the drugs and the difficulty of development is high, so that they are not commercialized.

In order to prepare an effective combination preparation, high bioavailability of active ingredients must be secured. The dissolution pattern of active ingredients from oral solid preparations is closely related to the bioavailability of the preparation, and high bioavailability presupposes a high dissolution rate. In general, the lower the particle size of the granules constituting the tablet, the more the dissolution rate has a tendency to be improved, but the lower the particle size of the granules, the lower the fluidity of the granules, and thus, the smaller the angle of repose. If the fluidity of the granules is low, there is a problem that productivity is lowered during the manufacture of tablets and capsules.

Therefore, in the technical field, there is a need for the development of a combination preparation having excellent pharmaceutical properties even in a combination preparation comprising losartan and ezetimibe, and continued research thereon is being conducted.

### [Disclosure]

### [Technical Problem]

The present inventors seek to confirm experimentally the dissolution correlation of each drug in a pharmaceutical combination preparation comprising ezetimibe and losartan as active ingredients, and provide a pharmaceutical combination preparation in which the dissolution between each drug is minimally affected in the pharmaceutical combination preparation comprising ezetimibe and losartan, thereby providing a pharmaceutical combination preparation having a similar pharmacokinetic parameter compared to a single ezetimibe preparation.

### [Technical Solution]

According to a first aspect of the present invention, the present invention provides a pharmaceutical combination preparation comprising ezetimibe or a pharmaceutically acceptable salt thereof, and losartan or a pharmaceutically acceptable salt thereof, wherein when the pharmaceutical combination preparation is dissolved in a solution of pH 4.0 to 5.0 for 10 minutes, the dissolved amount of the losartan is 5,700 µg or less, and the dissolved amount of the ezetimibe is 270 µg or more.

In an embodiment of the present invention, the dissolution in a solution of pH 4.0 to 5.0 is carried out under the conditions of a temperature of 37 °C and a paddle method of 50 rpm in 500 mL of acetate buffer according to United States Pharmacopoeia for the pharmaceutical combination preparation comprising 20 mg of ezetimibe and 183 mg of losartan.

In an embodiment of the present invention, when the pharmaceutical combination preparation is dissolved in a solution of pH 4.0 to 5.0 for 10 minutes, the dissolved amount of the losartan is 5,200 µg or less, and the dissolved amount of the ezetimibe is 300 µg or more.

In an embodiment of the present invention, the ezetimibe or a pharmaceutically acceptable salt thereof and the losartan or a pharmaceutically acceptable salt thereof are physically separated from each other.

In an embodiment of the present invention, the ezetimibe or a pharmaceutically acceptable salt thereof and the losartan or a pharmaceutically acceptable salt thereof are each present in a granular form.

In an embodiment of the present invention, the granule comprising the ezetimibe or a pharmaceutically acceptable salt thereof has a content of 15% by weight or less of granule having a diameter of more than 250 µm.

In an embodiment of the present invention, the granule comprising the losartan or a pharmaceutically acceptable salt thereof has a content of 35 to 55% by weight of granule having a diameter exceeding 250 µm, and a content of 10 to 30% by weight of granule having a diameter exceeding 500 µm.

In an embodiment of the present invention, the pharmaceutical combination preparation includes 5 to 10 mg of the ezetimibe or a pharmaceutically acceptable salt thereof as converted into the form of ezetimibe free acid, and includes 40 to 100 mg of the losartan or a pharmaceutically acceptable salt thereof as converted into the form of losartan free acid.

In an embodiment of the present invention, the granule comprising the ezetimibe or a pharmaceutically acceptable salt thereof further comprises a solubilizing agent.

In an embodiment of the present invention, the solubilizing agent is sodium lauryl sulfate.

In an embodiment of the present invention, the solubilizing agent is included in the granule comprising the ezetimibe or a pharmaceutically acceptable salt thereof, in an amount of 5 to 50 parts by weight, based on 100 parts by weight of ezetimibe.

In an embodiment of the present invention, the granule comprising the losartan or a pharmaceutically acceptable salt thereof further comprises 1 to 30 parts by weight of disintegrant, based on 100 parts by weight of losartan.

In an embodiment of the present invention, the pharmaceutical combination preparation is present in the form of a bilayer tablet consisting of: a first layer comprising the ezetimibe or a pharmaceutically acceptable salt thereof; and a second layer comprising the losartan or a pharmaceutically acceptable salt thereof.

According to a second aspect of the present invention, the present invention provides a pharmaceutical combination preparation having the form of a bilayer tablet consisting of: a first layer comprising ezetimibe or a pharmaceutically acceptable salt thereof, amlodipine or a pharmaceutically acceptable salt thereof, and rosuvastatin or a pharmaceutically acceptable salt thereof; and a second layer comprising losartan or a pharmaceutically acceptable salt thereof, wherein when the pharmaceutical combination preparation is dissolved in a solution of pH 4.0 to 5.0 for 10 minutes, the dissolved amount of the losartan is 5,700 µg or less, and the dissolved amount of the ezetimibe is 270 µg or more.

In an embodiment of the present invention, the ezetimibe or a pharmaceutically acceptable salt thereof is included in a granular form in the first layer, and the losartan or a pharmaceutically acceptable salt thereof is included in a granular form in the second layer.

### [Advantageous Effects]

The present invention provides a pharmaceutical combination preparation comprising ezetimibe and losartan, wherein the ezetimibe or a pharmaceutically acceptable salt thereof is present in a physically separated state from the losartan or a pharmaceutically acceptable salt thereof, and the dissolution rate of losartan is controlled, thereby being able to provide a pharmaceutical combination preparation in which the dissolution between each drug is minimally affected and being able to provide a pharmaceutical combination preparation of which the biological equivalence is secured.

In addition, if a pharmaceutical combination preparation comprising amlodipine and rosuvastatin in addition to the ezetimibe and losartan is provided, the ezetimibe is physically separated from the amlodipine, losartan, and rosuvastatin, respectively, and the dissolution rate of losartan is controlled, thereby being able to secure the biological equivalence of the pharmaceutical combination preparation.

### [Description of Drawings]

FIG. 1 is a graph showing the dissolved results of ezetimibe by pH (pH 1.2, 4.5, and 6.8) in the tablet according to Example 1.
FIG. 2 is a graph showing the dissolved results of ezetimibe at pH 4.5 in the tablets according to Example 1 and Comparative Example 1 and the capsules according to Example 3 and Comparative Example 2.
FIG. 3 is a graph showing the dissolved results of losartan at pH 4.5 in the tablets according to Example 1 and Comparative Example 1 and the capsules according to Example 3 and Comparative Example 2.
FIG. 4 is a graph showing the dissolved results of ezetimibe at pH 4.5 in the tablets according to Examples 1 and 2 and the tablets according to Comparative Examples 3 and 4.
FIG. 5 is a graph showing the dissolved results of losartan at pH 4.5 in the tablets according to Examples 1 and 2 and the tablets according to Comparative Examples 3 and 4.
FIG. 6 is a graph showing the dissolved results of ezetimibe at pH 4.5 in the tablets according to Examples 1 and 4.
FIG. 7 is a graph showing the dissolved results of losartan at pH 4.5 in the tablets according to Examples 1 and 4.
FIG. 8 is a graph showing the plasma concentration pattern of ezetimibe in the tablets according to Example 1, and Comparative Examples 1 and 3.

### [Best Mode]

The embodiments provided according to the present invention can be all achieved by the following description. It should be understood that the following description describes preferred embodiments of the present invention and the present invention is not necessarily limited thereto.

The present invention provides a pharmaceutical combination preparation comprising ezetimibe or a pharmaceutically acceptable salt thereof, and losartan or a pharmaceutically acceptable salt thereof.

The pharmaceutical combination preparation according to the present invention comprises ezetimibe or a pharmaceutically acceptable salt thereof as a first active ingredient. The ezetimibe serves to mainly inhibit cholesterol absorption for the treatment and prevention of arteriosclerosis. The daily dose of the ezetimibe or a pharmaceutically acceptable salt thereof is 5 mg to 15 mg. In one embodiment of the present invention, the pharmaceutical combination preparation includes 5 to 10 mg of the ezetimibe or a pharmaceutically acceptable salt thereof as converted into the form of ezetimibe free acid. Wherein, the free acid form of the active ingredient means the active ingredient itself to which other compounds such as base are not bound.

The pharmaceutical combination preparation according to the present invention may comprise losartan or a pharmaceutically acceptable salt thereof as a second active ingredient. Examples of the pharmaceutically acceptable salt of the losartan include, but are not necessarily limited to, losartan potassium salt. The losartan contributes to blocking the binding of angiotensin II, which is a vasoconstrictor substance, to its receptor to treat hypertension and heart failure, to treat ischemic peripheral circulatory disorder and myocardial ischemia (angina pectoris), to prevent the progression of heart failure after myocardial infarction, and to treat diabetic neuropathy, glaucoma, and the like. The daily dose of the losartan or a pharmaceutically acceptable salt thereof is 40 mg to 100 mg. In one embodiment of the present invention, the pharmaceutical combination preparation includes 40 to 100 mg of the losartan or a pharmaceutically acceptable salt thereof as converted into the form of losartan free acid.

In a pharmaceutical combination preparation comprising ezetimibe and losartan as active ingredients, the dissolution of losartan may affect the dissolution of ezetimibe. Since the influences do not appear equally under all conditions, it may be more important to select specific conditions to identify these influences. The present inventors have confirmed that, through the following examples and the like, when the pharmaceutical combination preparation comprising ezetimibe and losartan is dissolved in a solution of pH 4.0 to 5.0, the dissolved amount of ezetimibe largely depends on the dissolved amount of losartan, and the following provides a pharmaceutical combination preparation in which the dissolution between ezetimibe and losartan is minimally affected.

When the pharmaceutical combination preparation comprising ezetimibe and losartan according to the present invention is dissolved in a solution of pH 4.0 to 5.0 for 10 minutes, the dissolved amount of the losartan is 5,700 µg or less, preferably 5,450 µg or less, and more preferably 5,200 µg or less. When the pharmaceutical combination preparation is dissolved in a solution of pH 4.0 to 5.0 for 10 minutes, the dissolved amount of the ezetimibe is 270pg or more, preferably 285pg or more, and more preferably 300pg or more. The dissolution test is carried out under the conditions of a temperature of 37 °C and a paddle method of 50 rpm in 500 mL of acetate buffer according to United States Pharmacopoeia for the pharmaceutical combination preparation comprising 20 mg of ezetimibe and 183 mg of losartan, and the dissolved amount is an average value measured 5 times or more, preferably 10 times or more. The dissolved amount may be controlled by various methods such as the use of additives, the form of a granule or a preparation, and the like.

As described above, since losartan may affect the dissolution and saturation solubility of ezetimibe, it is preferable that the ezetimibe and losartan are present in a physically separated state to control the dissolution rate of losartan. In this case, the physically separated state means that a processing step for separating a reference active ingredient from other active ingredients is performed and the reference active ingredient is maintained in a state distinguished from other active ingredients in the final combination preparation obtained through this processing step, but the case where the ingredients themselves are mixed in a mixing process in advance without this processing step does not correspond to this meaning. The physically separated state can be achieved through, for example, physical separation using granulation, physical separation by multilayer tableting, physical separation by tablets each tableted separately, physical separation using a core-shell structure, etc. According to one embodiment of the present invention, in order to physically separate ezetimibe and losartan, the process of producing ezetimibe and losartan into a separate granular form may be performed and then mixed. At this time, in the case where the state in which the active ingredient (losartan) other than ezetimibe is not substantially present in the granules is maintained as in the processing step even after the completion of the combination preparation, even if there is an interface at which the ezetimibe is in contact with losartan on the surface of each of ezetimibe granules in the combination preparation, it can be said that the ezetimibe is present in a physically separated state from losartan in the combination preparation as described in the present invention. Likewise, in another embodiment, the combination preparation may be obtained by mixing rosuvastatin and amlodipine with the ezetimibe granule produced after granulation processing of ezetimibe, wherein in the case where the other active ingredients (rosuvastatin and amlodipine) are not substantially present in the ezetimibe granule, the ezetimibe may be regarded as being present in a physically separated state from rosuvastatin and amlodipine. In one embodiment of the present invention, the pharmaceutical combination preparation may be present in the form of a bilayer tablet consisting of: a first layer comprising the ezetimibe or a pharmaceutically acceptable salt thereof; and a second layer comprising the losartan or a pharmaceutically acceptable salt thereof.

The method for producing an ezetimibe granule is not especially limited, but it may be preferable to produce granules in a wet state. Ezetimibe is a poorly soluble drug having a low saturation solubility of about 1 ppm under acidic to weakly basic conditions such as body fluids. The saturation solubility and the dissolution rate from the start of dissolution to the point of saturation may be an important indicator for evaluating the bioavailability of the poorly soluble drug, and wet granules may reach saturation solubility at a faster rate, so that high bioavailability may be secured for the poorly soluble drug ezetimibe. The method for producing a losartan granule is not especially limited, but it may be preferable to produce granules by a compression granulation method. When ezetimibe granules are sieved through sieve opening size of 250 µm (Size 60), the content of granules (residues) having a diameter of more than 250 µm may be 15% by weight or less, preferably 10% by weight or less. When losartan granules are sieved through sieve opening size of 250 µm, the content of granules (residues) having a diameter of more than 250 µm may be 35 to 55% by weight, preferably 40 to 50% by weight, and when they are sieved through sieve opening size of 500 µm (Size 35), the content of granules (residues) having a diameter of more than 500µm may be 10 to 30% by weight, preferably 15 to 25% by weight. If the ezetimibe granules and losartan granules are adjusted within the above ranges, the dissolved amounts of ezetimibe granules and losartan can be adjusted at an appropriate level.

The combination preparation of the present invention may further comprise one or more pharmaceutically acceptable additives necessary for preparation in addition to the active ingredients above. Specifically, the ezetimibe granule part or the losartan granule part may comprise one or more pharmaceutically acceptable additives selected from the group consisting of excipients, binders, and disintegrants. According to one embodiment of the present invention, the excipient may be included in the ezetimibe granule part in an amount of 50 to 5,000 parts by weight, preferably 500 to 4,000 parts by weight, more preferably 1,000 to 3,000 parts by weight based on 100 parts by weight of ezetimibe. The binder may be included in the ezetimibe granule part in an amount of 10 to 1,000 parts by weight, preferably 20 to 500 parts by weight, more preferably 30 to 100 parts by weight based on 100 parts by weight of ezetimibe. The disintegrant may be included in the ezetimibe granule part in an amount of 10 to 1,000 parts by weight, preferably 20 to 500 parts by weight, more preferably 30 to 300 parts by weight based on 100 parts by weight of ezetimibe. According to one embodiment of the present invention, the excipient may be included in the losartan granule part in an amount of 50 to 1,000 parts by weight, preferably 100 to 500 parts by weight, more preferably 150 to 300 parts by weight based on 100 parts by weight of losartan. Since the dissolution of losartan may affect the dissolution of ezetimibe, the content of the disintegrant in the losartan granule part may be an important element in preparing the pharmaceutical combination preparation according to the present invention. In one embodiment of the present invention, the disintegrant may be included in an amount of 1 to 30 parts by weight, preferably 10 to 25 parts by weight based on 100 parts by weight of losartan. If the disintegrant is included in an amount of less than 1 part by weight, the dissolution of losartan may be lowered, and if the disintegrant is included in an amount of more than 30 parts by weight, the dissolution of ezetimibe may be lowered due to the dissolution of losartan. The ezetimibe granule part or losartan granule part may be mixed with glidants after preparation of the granules. According to one embodiment of the present invention, the glidant may be mixed with the ezetimibe granule part in an amount of 5 to 300 parts by weight, preferably 10 to 200 parts by weight, more preferably 15 to 100 parts by weight based on 100 parts by weight of ezetimibe, and may be mixed with the losartan granule part in an amount of 0.1 to 20 parts by weight, preferably 0.5 to 15 parts by weight, more preferably 1 to 10 parts by weight based on 100 parts by weight of losartan.

The kind and content of such additives can be appropriately selected according to the type of the specific formulation to be prepared by one skilled in the art. For example, the excipient is selected from the group consisting of, but is not limited to, lactose, starch, mannitol, microcrystalline cellulose, carboxymethylcellulose, and combinations thereof; the binder is selected from the group consisting of, but is not limited to, povidone, hypromellose, hydroxypropylcellulose, copovidone, and combinations thereof; the disintegrant is selected from the group consisting of, but is not limited to, crospovidone, croscarmellose sodium, sodium starch glycolate, low-substituted hydroxypropylcellulose, and combinations thereof; and the glidant is selected from the group consisting of, but is not limited to, magnesium stearate, talc, light anhydrous silicic acid, sodium stearyl fumarate, and combinations thereof.

Since ezetimibe is a poorly soluble drug as described above, the ezetimibe granule part may further comprise a solubilizing agent. In one embodiment of the present invention, the solubilizing agent may be included in the ezetimibe granule part in an amount of 5 to 50 parts by weight, preferably 10 to 40 parts by weight, more preferably 15 to 30 parts by weight based on 100 parts by weight of ezetimibe. If the amount of the solubilizing agent is less than 5 parts by weight, the dissolution rate of ezetimibe is remarkably lowered, whereas, if the amount of the solubilizing agent is more than 50 parts by weight, the improved effect of the dissolution rate of ezetimibe due to the increase of the solubilizing agent is insignificant. In addition, if the solubilizing agent is used in an excessive amount, the stability of the ezetimibe granules may be lowered. Sodium lauryl sulfate (SLS) may be preferably used as the solubilizing agent.

The pharmaceutical combination preparation according to the present invention may further comprise the ingredient selected from the group consisting of amlodipine or a pharmaceutically acceptable salt thereof, rosuvastatin or a pharmaceutically acceptable salt thereof, and combinations thereof.

The pharmaceutical combination preparation according to the present invention may comprise amlodipine or pharmaceutically acceptable salt thereof as a third active ingredient. Examples of the pharmaceutically acceptable salt of amlodipine include, but is necessarily limited to, hydrochloride, hydrobromide, sulfate, phosphate, acetate, malate, fumarate, lactate, tartrate, citrate, gluconate, besilate, and camsylate, preferably amlodipine besilate salt and amlodipine camsylate salt. In addition, the amlodipine of the present invention includes amlodipine racemates and (S)-amlodipine. The amlodipine is used in the treatment of cardiovascular diseases such as angina pectoris, hypertension, and congestive heart failure by blocking the calcium channel. The daily dose of the amlodipine or a pharmaceutically acceptable salt thereof is 5 mg to 20 mg.

The pharmaceutical combination preparation according to the present invention comprises rosuvastatin or a pharmaceutically acceptable salt thereof as a fourth active ingredient. Examples of the pharmaceutically acceptable salt of rosuvastatin include, but are not limited to, calcium salt, magnesium salt, strontium salt, etc., and preferably rosuvastatin calcium salt. The rosuvastatin contributes to the treatment of dyslipidemia by inhibiting HMG-CoA reductase, which is essential for the synthesis of cholesterol, thereby lowering the blood LDL cholesterol levels, while increasing the HDL cholesterol levels. The daily dose of the rosuvastatin or a pharmaceutically acceptable salt thereof is 10 mg to 20 mg.

The ezetimibe is present in a physically separated state from the rosuvastatin, amlodipine, and losartan, respectively. Wherein, the physically separated state follows the above description. If the pharmaceutical combination preparation comprises all of ezetimibe, rosuvastatin, amlodipine, and losartan as active ingredients, the pharmaceutical combination preparation may be present in the form of a bilayer tablet consisting of: a first layer comprising the ezetimibe or a pharmaceutically acceptable salt thereof, the rosuvastatin or a pharmaceutically acceptable salt thereof, and the amlodipine or a pharmaceutically acceptable salt thereof; and a second layer comprising the losartan or a pharmaceutically acceptable salt thereof. The ezetimibe or a pharmaceutically acceptable salt thereof in the first layer is present in admixture with the rosuvastatin or a pharmaceutically acceptable salt thereof, and the amlodipine or a pharmaceutically acceptable salt thereof, in a granular form. The losartan or a pharmaceutically acceptable salt thereof in the second layer is present in a granular form. The first and second layers in the bilayer tablet may comprise one or more pharmaceutically acceptable additives selected from the group consisting of excipients, binders, disintegrants, and glidants.

The present invention provides a method of preparing a pharmaceutical combination preparation comprising ezetimibe or a pharmaceutically acceptable salt thereof, and losartan or a pharmaceutically acceptable salt thereof.

The method of preparing a pharmaceutical combination preparation according to the present invention comprises the steps of: i) producing an ezetimibe granule comprising ezetimibe or a pharmaceutically acceptable salt thereof; ii) producing a losartan granule comprising losartan or a pharmaceutically acceptable salt thereof; and iii) controlling the dissolved amount of ezetimibe or losartan. The step iii) is to control the dissolved amount of ezetimibe or losartan in accordance with the above-mentioned criteria, and the dissolved amount of ezetimibe or losartan may be controlled by the use of additives, the form of a granule or a preparation, and the like. The details of the preparation method are supplemented by the above-mentioned descriptions and the following examples.

Hereinafter, preferred examples will be provided to help to understand the present invention, but the following examples are provided not to limit the present invention but to facilitate the understanding of the present invention.

### Examples

### Example 1: Preparation of Bilayer Tablet Comprising Ezetimibe and Losartan

A coated bilayer tablet comprising ezetimibe and losartan as active ingredients was prepared according to the composition shown in Table 1 below.

Specifically, ezetimibe was placed in a fluidized bed granulator together with lactose hydrate, microcrystalline cellulose, croscarmellose sodium, and sodium lauryl sulfate, and was mixed for 3 minutes. A binder solution in which povidone was dissolved in water was added thereto, and the transfer pump was set at 2 rpm and combined to perform granulation for about 50 minutes. The resulting product was dried in a fluidized bed drier at 45 °C, and then was sieved through sieve opening size of 0.6 mm to produce an ezetimibe granule. At this time, the ezetimibe granule had a content of 5% by weight of granule having a diameter of more than 250 µm.

Losartan potassium was placed in a mixer together with lactose hydrate, microcrystalline cellulose, and crospovidone, and was mixed for 20 minutes. The resulting product was pressed under the conditions of an oil pressure of 2 MPa, a feeder speed of 5 rpm, and a roller speed of 1 rpm using a roller compressor (TF-1-A60, Freund vector) to form flakes, and then a losartan granule was produced by sizing through sieve opening size of 0.8 mm. At this time, the losartan granule had a content of 45% by weight of granule having a diameter of more than 250 µm, and had a content of 20% by weight of granule having a diameter of more than 500 µm.

Magnesium stearate was added to the ezetimibe granule and further mixed in a mixer for 5 minutes to prepare an upper layer mixture of the bilayer tablet. In addition, magnesium stearate was added to the losartan granule and further mixed in a mixer for 5 minutes to prepare an lower layer mixture of the bilayer tablet. The upper layer and lower layer mixtures were pressurized with a tableting machine (Autotab-200TR, ichihashi seiki) to prepare a bilayer tablet having a hardness of about 12 kp.

The bilayer tablet was coated using a coating machine (SFC-30F, Sejong) according to the coating layer prescription shown in Table 1 below.

### Example 2: Preparation of Bilayer Tablet Comprising Ezetimibe and Losartan

A coated bilayer tablet comprising ezetimibe and losartan as active ingredients was prepared according to the composition shown in Table 1 below in the same manner as in Example 1.

### Example 3: Preparation of Capsule Containing Mini Tablet Comprising Ezetimibe and Losartan

A capsule containing a coated mini tablet each comprising ezetimibe and losartan as an active ingredient was prepared according to the composition shown in Table 1 below.

Specifically, an ezetimibe granule and a losartan granule were prepared in the same manner as in Example 1. A losartan-containing mini tablet (303 mg/30T) and an ezetimibe-containing mini tablet (157 mg/10T), which have a diameter of 2 mm and a hardness of about 1.5 kp, were respectively prepared from each granule with a tableting machine. The losartan-containing mini tablet and the ezetimibe-containing mini tablet were respectively coated using a fluidized bed coating machine according to the coating layer prescription shown in Table 1 and filled into size 0 hard capsules.

### Example 4: Preparation of Bilayer Tablet Comprising Ezetimibe, Rosuvastatin, Amlodipine, and Losartan

A coated bilayer tablet comprising ezetimibe, rosuvastatin, amlodipine, and losartan as active ingredients was prepared according to the composition shown in Table 1 below.

Specifically, a losartan granule was produced in the same manner as in Example 1, and magnesium stearate was mixed with the losartan granule to prepare an upper layer mixture.

In addition, an ezetimibe granule was produced in the same manner as in Example 1, and then the ezetimibe granule was placed in a mixer together with a mixture having the composition described in the mixing part of Table 1 and mixed for 15 minutes. Thereafter, magnesium stearate was added thereto and further mixed in a mixer for 5 minutes to prepare an lower layer mixture. The upper layer and lower layer mixtures were pressurized with a tableting machine to prepare a bilayer tablet having a hardness of about 20 kp.

The bilayer tablet was coated using a coating machine according to the coating layer prescription shown in Table 1 below.

The specific compositions of the pharmaceutical combination preparations according to Examples 1 to 4 are shown in Table 1 below.

**[Table 1]**

| **Classific ation** | **Ingredient** | **Example 1 (mg/T)** | **Example 2 (mg/T)** | **Example 3 (mg/C)** | | **Example 4 (mg/T)** |
|---|---|---|---|---|---|---|
| Losartan granule | Losartan potassium (as losartan) | 100.0 | 100.0 | 100.0 | - | 100.0 |
| | | (91.5) | (91.5) | (91.5) | | (91.5) |
| | Microcrystalline | 125.0 | 120.0 | 125.0 | - | 125.0 |
| | cellulose (Avicel PH101) | | | | | |
| | Lactose hydrate (#200) | 60.0 | 60.0 | 60.0 | - | 60.0 |
| | Crospovidone (XL-10) | 15.0 | 20.0 | 15.0 | - | 15.0 |
| Postmixtu re | Magnesium stearate | 3.0 | 3.0 | 3.0 | - | 3.0 |
| **Total mass** | | **303.0** | **303.0** | **303.0** | **-** | **303.0** |
| Ezetimibe granule | Ezetimibe | 10.0 | 10.0 | - | 10.0 | 10.0 |
| | Microcrystalline cellulose (Avicel PH101) | 90.0 | 90.0 | - | 90.0 | 90.0 |
| | Lactose hydrate (#200) | 35.0 | 35.0 | - | 35.0 | 35.0 |
| | Croscarmellose sodium | 15.0 | 15.0 | - | 15.0 | 15.0 |
| | Sodium lauryl sulfate | 2.0 | 2.0 | - | 2.0 | 2.0 |
| | Povidone (k-30) | 5.0 | 5.0 | - | 5.0 | 5.0 |
| | Purified water* | (70.0) | (70.0) | - | (70.0) | (70.0) |
| Postmixtu re | Magnesium stearate | 2.0 | 2.0 | - | 2.0 | - |
| **Total mass** | | **159.0** | **159.0** | **-** | **159.0** | **157.0** |
| Mixing part | Amlodipine besilate (as amlodipine) | - | - | - | - | 6.94 (5.0) |
| | Rosuvastatin calcium (as rosuvastatin) | - | - | - | - | 20.8 (20.0) |
| | Mannitol (SD200) | - | - | - | - | 120.0 |
| | Lactose hydrate (supertab) | - | - | - | - | 120.0 |
| | Microcrystalline cellulose (Avicel PH101) | - | - | - | - | 30.0 |
| | Crospovidone (XL-10) | - | - | - | - | 24.0 |
| | Magnesium stearate | - | - | - | - | 4.0 |
| **Total mass of uncoated tablet** | | **462.0** | **462.0** | **303.0** | **159.0** | **785.74** |
| Coating layer | Hydroxypropyl cellulose | 6.4 | 6.4 | 4.3 | 10.9 | 2.1 |
| | Polyethylene glycol | 0.9 | 0.9 | 0.6 | 1.6 | 0.3 |
| | Talc | 0.2 | 0.2 | 0.2 | 0.4 | 0.1 |
| | Titanium oxide | 0.9 | 0.9 | 0.6 | 1.5 | 0.3 |
| | Red iron oxide | 0.4 | 0.4 | 0.2 | 0.6 | 0.1 |
| | Purified water* | (80.6) | (80.6) | (52.8) | (137.0) | (27.7) |
| **Total mass of coated tablet** | | **470.8** | **470.8** | **308.9** | **161.9** | **470.8** |

| | | | | | | |
|---|---|---|---|---|---|---|
| * Purified water is removed during the process. | | | | | | |

### Comparative Examples 1: Preparation of Tablet Comprising Ezetimibe

A coated tablet comprising ezetimibe as an active ingredient was prepared according to the composition shown in Table 2 below.

Specifically, a mixture comprising an ezetimibe granule was prepared in the same manner as in Example 1 and pressurized with a tableting machine to prepare a tablet having a hardness of about 7 kp.

The tablet was coated using a coating machine according to the coating layer prescription shown in Table 2 below.

### Comparative Examples 2: Preparation of Capsule Comprising Ezetimibe Mini Tablet and Losartan Granule

A coated mini tablet (157 mg/10T) comprising the losartan granule obtained in Example 1 and the ezetimibe obtained in Example 3 was filled into a size 0 hard capsule.

### Comparative Examples 3 and 4: Preparation of Bilayer Tablet Comprising Ezetimibe and Losartan

A coated bilayer tablet comprising ezetimibe and losartan as active ingredients was prepared according to the composition shown in Table 2 below in the same manner as in Example 1.

The specific compositions of the pharmaceutical combination preparations according to Comparative Examples 1, 3, and 4 are shown in Table 2 below.

**[Table 2]**

| **Classification** | **Ingredient** | **Comparative Example 1 (mg/T)** | **Comparative Example 3 (mg/T)** | **Comparative Example 4 (mg/T)** |
|---|---|---|---|---|
| Losartan granule | Losartan potassium (as losartan) | - | 100.0 (91.5) | 100.0 (91.5) |
| | Microcrystalline cellulose (Avicel PH101) | - | 110.0 | 95.0 |
| | Lactose hydrate (#200) | - | 60.0 | 60.0 |
| | Crospovidone (XL-10) | - | 30.0 | 45.0 |
| Postmixture | Magnesium stearate | - | 3.0 | 3.0 |
| Total mass | | - | 303.0 | 303.0 |
| Ezetimibe granule | Ezetimibe | 10.0 | 10.0 | 10.0 |
| | Microcrystalline cellulose (Avicel PH101) | 90.0 | 90.0 | 90.0 |
| | Lactose hydrate (#200) | 35.0 | 35.0 | 35.0 |
| | Croscarmellose sodium | 15.0 | 15.0 | 15.0 |
| | Sodium lauryl sulfate | 2.0 | 2.0 | 2.0 |
| | Povidone (k-30) | 5.0 | 5.0 | 5.0 |
| | Purified water* | (70.0) | (70.0) | (70.0) |
| Postmixture | Magnesium stearate | 2.0 | 2.0 | 2.0 |
| **Total mass** | | **159.0** | **159.0** | **159.0** |
| **Total mass of uncoated tablet** | | **159.0** | **462.0** | **462.0** |
| Coating layer | Hydroxypropyl cellulose | 2.2 | 6.4 | 6.4 |
| | Polyethylene glycol | 0.3 | 0.9 | 0.9 |
| | Talc | 0.1 | 0.2 | 0.2 |
| | Titanium oxide | 0.3 | 0.9 | 0.9 |
| | Red iron oxide | 0.1 | 0.4 | 0.4 |
| | Purified water* | (27.7) | (80.6) | (80.6) |
| **Total mass of coated tablet** | | **162.0** | **470.8** | **470.8** |

| | | | | |
|---|---|---|---|---|
| * Purified water is removed during the process. | | | | |

### Test Example

### Test Example 1: Dissolution Test

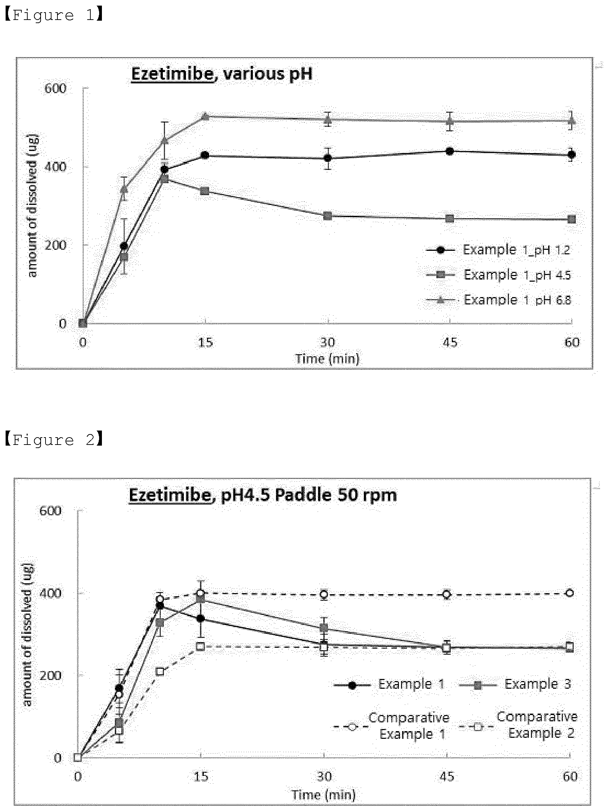

Under the following dissolution conditions and analytical conditions, the dissolution rates of ezetimibe and losartan in the tablets or capsules according to Examples 1 to 4 and Comparative Examples 1 to 4 were respectively measured, and the results are shown in Figs. 1 to 7.

### <Dissolution Conditions>

Dissolution medium: Two tablets or two capsules (20 mg as ezetimibe, 183 mg as losartan) are taken and tested in 500 mL of the following solution.
- pH 1.2: Simulated gastric fluid USP without enzyme
- pH 4.5: Acetic acid buffer USP
- pH 6.8: Simulated intestinal fluid USP without enzyme Device: Paddle method, 50±2 rpm

Temperature: 37±0.5 °C

### Dissolution time: 5, 10, 15, 30, 45, 60 minutes

(After 45 minutes, the paddle speed was changed to 150 rpm and stirred for 15 minutes, and then the dissolution medium was taken at 60 minutes.)

### <Analytical Conditions>

Column: A stainless steel tube having an inner diameter of 4.6 mm and a length of 15 cm was packed with 3 µm of an octadecyl silylated silica gel for liquid chromatography.

Mobile phase: * 9 mM sodium hexanesulfonate/0.13% (v/v) phosphoric acid : acetonitrile (52:48, v/v)

(* 9 mM sodium hexanesulfonate/0.13% (v/v) phosphoric acid : 1.86 g of sodium hexanesulfonate (sodium 1-hexanesulfonate monohydrate) is placed in a 1 L flask and 1.3 mL of phosphoric acid is carefully added thereto. Purified water is added thereto, and they are dissolved, diluted, and well mixed.
Detector: Ultraviolet absorption spectrophotometer (measurement wavelength 254 nm)
Flow rate: 1.3 mL/min
Injection volume: 10 µL
Column temperature: 45 °C

FIG. 1 shows the dissolved results of ezetimibe by pH in the tablet according to Example 1. According to FIG. 1, it was confirmed that the dissolved amount of the main ingredient is maintained when the tablet according to Example 1 is dissolved at pH 1.2 and pH 6.8, whereas when it is dissolved at pH 4.5, which is between pH 1.2 and pH 6.8, for about 10 minutes, the dissolved amount of the main ingredient gradually decreases after reaching the maximum value to remain lower than the maximum value.

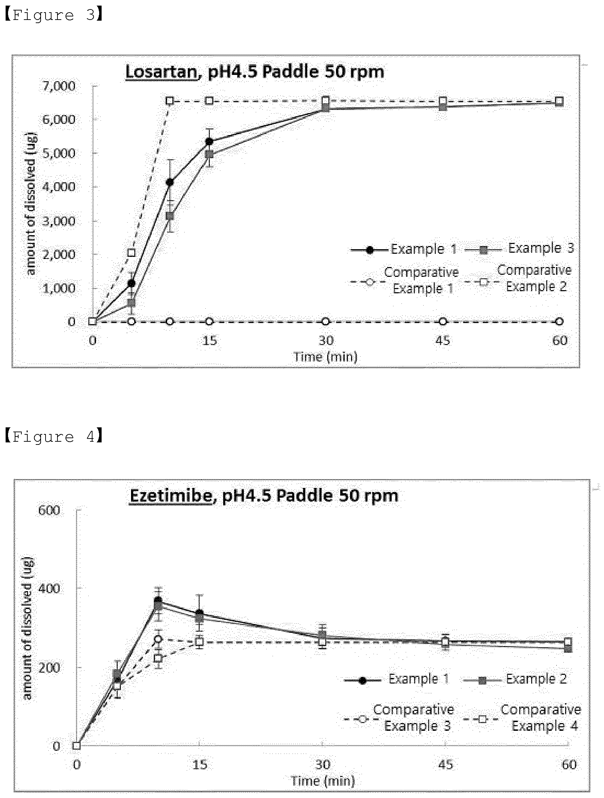

FIGS. 2 and 3 show the dissolved results of ezetimibe and losartan at pH 4.5 in the tablets according to Example 1 and Comparative Example 1 and the capsules according to Example 3 and Comparative Example 2. First, the dissolution patterns of the tablet according to Example 1 comprising ezetimibe and losartan and the tablet according to Comparative Example 1 comprising only ezetimibe prepared in the same manner without losartan were compared. According to FIG. 2, it was confirmed that in the case of the tablet according to Comparative Example 1 that does not comprise losartan, the dissolved amount of ezetimibe at pH 4.5 is maintained at the maximum value. Ezetimibe is a poorly soluble drug and is known to exhibit low solubility in dissolution medium containing no surfactant, but the deposition of ezetimibe again after dissolution is considered to be an unusual phenomenon and it is determined that this is because losartan affects the dissolution and saturation solubility of ezetimibe.

Subsequently, although the ezetimibe prepared in the same manner was included, the capsule according to Example 3 having a controlled dissolution rate by including losartan in the form of a coated mini tablet and the capsule according to Comparative Example 2 having a rapid dissolution by including losartan in the form of a granule, were compared. According to FIGS. 2 and 3, it was confirmed that the initial dissolution rate and saturation solubility of ezetimibe were low when the dissolution of losartan was rapid, as in the capsule according to Comparative Example 2. Through these results, it is determined that losartan affects the dissolution pattern of ezetimibe, as in the previous comparison of the tablet according to Example 1 and the tablet according to Comparative Example 1.

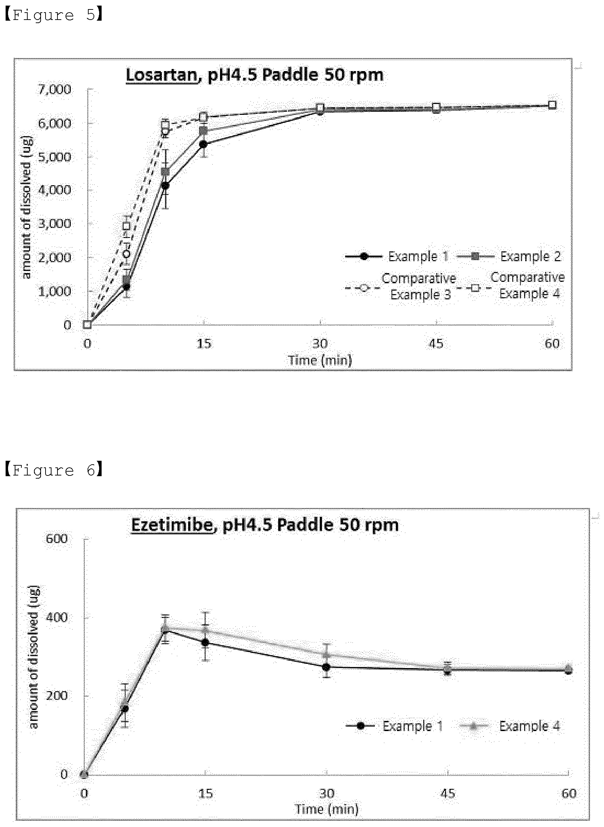

Figs. 4 and 5 show the dissolved results of ezetimibe and losartan at pH 4.5 in the tablets according to Examples 1 and 2 and the tablets according to Comparative Examples 3 and 4. The tablets according to Examples 1 and 2 and the tablets according to Comparative Examples 3 and 4 differ in the amount of disintegrants in the losartan layer, respectively. It was confirmed that if the disintegrant was included in the losartan layer in an amount of 15 mg and 20 mg, respectively, as in the tablets according to Examples 1 and 2, the dissolution patterns of losartan and ezetimibe appeared similarly. On the contrary, it was confirmed that if the disintegrant was included in the losartan layer in an amount of 30 mg and 45 mg, respectively, as in the tablets according to Comparative Examples 3 and 4, compared to the tablets according to Examples 1 and 2, the dissolution rate of losartan was increased, but the maximum dissolved amount of ezetimibe was small.

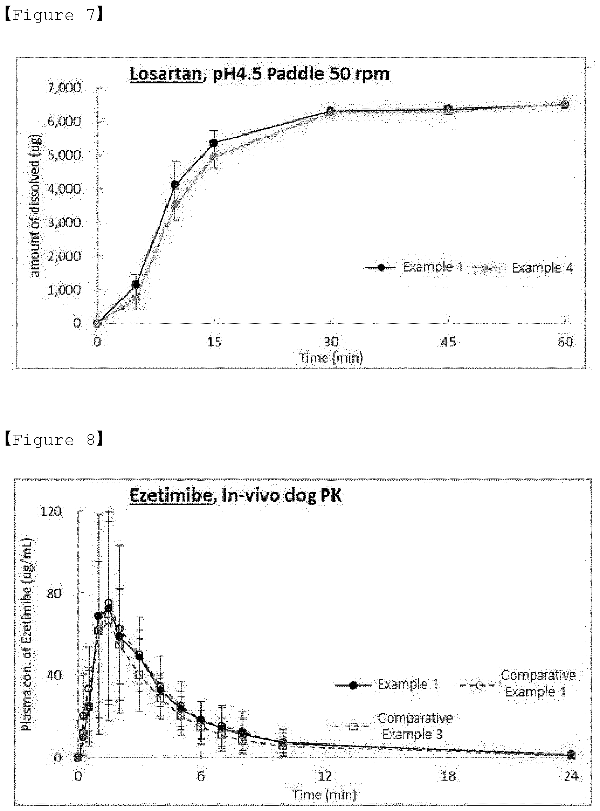

FIGS. 6 and 7 show the dissolved results of ezetimibe and losartan at pH 4.5 in the tablets according to Examples 1 and 4. According to FIGS. 6 and 7, it was confirmed that even if amalodipine and rosuvastatin are mixed with the ezetimibe granule as in the tablet according to Example 4, the maximum dissolved amount of ezetimibe was not significantly different compared to the tablet according to Example 1. Through these results, it is determined that the dissolution pattern of ezetimibe is directly affected by losartan rather than amlodipine and rosuvastatin.

### Test Example 2: Pharmacokinetic Evaluation of Ezetimibe

For the compositions of Example 1, Comparative Example 1, and Comparative Example 3, pharmacokinetic parameters of ezetimibe were evaluated in beagle dogs. Eighteen beagle dogs (Male, 20 months of age, 12±2 kg) were randomly assigned to three groups and a 3-way crossover study was conducted for six beagle dogs per group. To each group of beagle dogs pre-fasted for 14 hours the day before, Example 1, Comparative Example 1, and Comparative Example 3 were orally administered, respectively, and then 40 mL of water was forcibly administered. Blood collection was performed by taking 2 mL of blood from the cephalic vein in a tube containing anticoagulant (1,000 IU/mL, heparin 5 µL) before oral administration (0 hour) and at 0.25, 0.5, 1, 1.5, 2, 3, 4, 5, 6, 7, 8, 10, and 24 hours after oral administration. Blood samples were centrifuged (12,000 rpm, 2 minutes, Eppendrof) to separate plasma and stored in a -20 °C freezer.

For analysis, 15 µL of β-glucuronidase (Helix pomatia) is added to 100 µL of plasma and mixed for 30 seconds. 100 µL of distilled water is added thereto and mixed for 30 seconds, and then reacted at 50 °C for 1 hour. 15 µL of the internal standard solution (Ezetimibe-d4, 50 ppb) is added to the plasma after 1 hour enzymatic reaction and mixed for 30 seconds. 1 mL of methyl tert-butyl ether (MTBE) is added thereto and mixed for 10 minutes, and then centrifuged (15,000 rpm, 3 minutes). The supernatant is taken and evaporated with a 45 °C nitrogen evaporator. The residue is redissolved in 1 mL of 50% ACN, and then analyzed using liquid chromatography-mass spectrometry (LC-MS) .

### <Analytical Conditions>

Device used: Xevo TQ-S (Waters)
Ionization mode: [M-H]+ (Negative)
Column: CHS C18 (2.1*150 mm, 1.8 µm, Waters)
Column temperature: 35 °C
Mobile phase: ACN/2mM ammonium acetate = 70/30 (v/v)
Flow rate: 0.18 mL/min
Injection volume: 5 µL

The results are shown in Table 3 below and FIG. 8. shows the recording of the arithmetic mean plasma concentration of ezetimibe (ng/ml) versus time (hr) on a linear scale.

**[Table 3]**

| | **Pharmacokinetic Evaluation of Ezetimibe** | | | | |
|---|---|---|---|---|---|
| **Parameter** | **Example 1 (T1)** | **Comparative Examples 3 (T2)** | **Comparative Examples 1 (R)** | **T1/R** | **T2/R** |
| AUC_{O-24} (ng ·hr/mL) | 347.01 ± 109.51 | 299.81 ± 115.12 | 360.62 ± 148.25 | 0.962 | 0.831 |
| Cₘₐₓ (ng/mL) | 87.68 ± 47.29 | 78.74 ± 50.94 | 86.79 ± 44.15 | 1. 010 | 0.907 |

As shown in Table 3 and FIG. 8, it was confirmed that compared to the tablet according to Comparative Example 1, the tablet according to Example 1 had the equivalent level within ±10% of AUC and Cₘₐₓ, but the tablet according to Comparative Example 3 had the unequal level close to the lower limit criteria.

Taking the results of Test Examples 1 and 2 together, the dissolution pattern of ezetimibe was changed according to the dissolution rate of losartan, and thus the change in pharmacokinetic parameters was confirmed. Therefore, it was confirmed that the dissolution rate of losartan should be controlled at pH 4.5 in developing a pharmaceutical combination preparation comprising ezetimibe and losartan.

It should be appreciated that all the simple modifications and variations of the present invention are within the scope of the present invention, and the specific scope of the present invention to be protected will be defined by the appended claims.

## Claims

1. A pharmaceutical combination preparation comprising ezetimibe or a pharmaceutically acceptable salt thereof, and losartan or a pharmaceutically acceptable salt thereof, wherein when the pharmaceutical combination preparation is dissolved in a solution of pH 4.0 to 5.0 for 10 minutes, the dissolved amount of the losartan is 5,700 µg or less, and the dissolved amount of the ezetimibe is 270 µg or more.

2. The pharmaceutical combination preparation according to claim 1, wherein the dissolution in a solution of pH 4.0 to 5.0 is carried out under the conditions of a temperature of 37 °C and a paddle method of 50 rpm in 500 mL of acetate buffer according to United States Pharmacopoeia for the pharmaceutical combination preparation comprising 20 mg of ezetimibe and 183 mg of losartan.

3. The pharmaceutical combination preparation according to claim 1, wherein when the pharmaceutical combination preparation is dissolved in a solution of pH 4.0 to 5.0 for 10 minutes, the dissolved amount of losartan is 5,200 µg or less, and the dissolved amount of the ezetimibe is 300 µg or more.

4. The pharmaceutical combination preparation according to claim 1, wherein the ezetimibe or a pharmaceutically acceptable salt thereof and the losartan or a pharmaceutically acceptable salt thereof are physically separated from each other.

5. The pharmaceutical combination preparation according to claim 4, wherein the ezetimibe or a pharmaceutically acceptable salt thereof and the losartan or a pharmaceutically acceptable salt thereof are each present in a granular form.

6. The pharmaceutical combination preparation according to claim 5, wherein the granule comprising the ezetimibe or a pharmaceutically acceptable salt thereof has a content of 15% by weight or less of granule having a diameter of more than 250 µm.

7. The pharmaceutical combination preparation according to claim 5, wherein the granule comprising the losartan or a pharmaceutically acceptable salt thereof has a content of 35 to 55% by weight of granule having a diameter of more than 250 µm, and has a content of 10 to 30% by weight of granule having a diameter of more than 500 µm.

8. The pharmaceutical combination preparation according to claim 1, wherein the pharmaceutical combination preparation includes 5 to 10 mg of the ezetimibe or a pharmaceutically acceptable salt thereof as converted into the form of ezetimibe free acid, and includes 40 to 100 mg of the losartan or a pharmaceutically acceptable salt thereof as converted into the form of losartan free acid.

9. The pharmaceutical combination preparation according to claim 5, wherein the granule comprising the ezetimibe or a pharmaceutically acceptable salt thereof further comprises a solubilizing agent.

10. The pharmaceutical combination preparation according to claim 9, wherein the solubilizing agent is sodium lauryl sulfate.

11. The pharmaceutical combination preparation according to claim 9, wherein the solubilizing agent is included in the granule comprising the ezetimibe or a pharmaceutically acceptable salt thereof, in an amount of 5 to 50 parts by weight, based on 100 parts by weight of ezetimibe.

12. The pharmaceutical combination preparation according to claim 5, wherein the granule comprising the losartan or a pharmaceutically acceptable salt thereof further comprises 1 to 30 parts by weight of disintegrant, based on 100 parts by weight of losartan.

13. The pharmaceutical combination preparation according to claim 1, wherein the pharmaceutical combination preparation is present in the form of a bilayer tablet consisting of:
a first layer comprising the ezetimibe or a pharmaceutically acceptable salt thereof; and
a second layer comprising the losartan or a pharmaceutically acceptable salt thereof.

14. A pharmaceutical combination preparation having the form of a bilayer tablet consisting of:
a first layer comprising ezetimibe or a pharmaceutically acceptable salt thereof, amlodipine or a pharmaceutically acceptable salt thereof, and rosuvastatin or a pharmaceutically acceptable salt thereof; and
a second layer comprising losartan or a pharmaceutically acceptable salt thereof,
wherein when the pharmaceutical combination preparation is dissolved in a solution of pH 4.0 to 5.0 for 10 minutes, the dissolved amount of the losartan is 5,700 µg or less, and the dissolved amount of the ezetimibe is 270 µg or more.

15. The pharmaceutical combination preparation according to claim 14, wherein the ezetimibe or a pharmaceutically acceptable salt thereof is included in a granular form in the first layer, and the losartan or a pharmaceutically acceptable salt thereof is included in a granular form in the second layer.
